Europäisches Patentamt

European Patent Office

Office européen des brevets

(11) Publication number: **0 253 410 B1**

(12) **EUROPEAN PATENT SPECIFICATION**

(45) Date of publication of patent specification: **01.04.92**    (51) Int. Cl.⁵: **C07C 21/18**, C07C 17/00

(21) Application number: **87110386.7**

(22) Date of filing: **17.07.87**

⑤④ Process for preparing hydrogen containing fluoroethylenes and chlorofluoroethylenes from chlorofluoroethanes.

(30) Priority: **18.07.86 IT 2117186**

(43) Date of publication of application:
**20.01.88 Bulletin 88/03**

(45) Publication of the grant of the patent:
**01.04.92 Bulletin 92/14**

(84) Designated Contracting States:
**BE DE ES FR GB IT NL SE**

(56) References cited:
EP-A- 0 053 657
US-A- 2 704 775
US-A- 2 864 873
US-A- 3 505 417
US-A- 3 636 173

**CHEMICAL ABSTRACTS, vol. 104, no. 13, 31st March 1982, page 667, abstract no. 109005x, Columbus Ohio, US; & JP-A-60 185 734**

(73) Proprietor: **AUSIMONT S.p.A.**
**Foro Buonaparte, 31**
**I-20121 Milano(IT)**

(72) Inventor: **Gervasutti, Claudio**
**2/4, via Paolo Sarpi**
**I-30175 Mestre-Venezia(IT)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

**Description**

This invention relates to a process for preparing hydrogen-containing fluoroethylenes and chlorofluoroethylenes by hydrogenolysis of certain chlorofluoroethanes.

Fluoroethylenes and chlorofluoroethylenes are halogenated olefins well known in literature and they are advantageously utilized as intermediates in the preparation of fluoroplastomers, fluoroelastomers and as comonomers in the preparation of fluorinated copolymers.

Fluorine-containing olefins are generally prepared by dehydrohalogenation or dehalogenation of the corresponding haloalkanes in the liquid phase, according to well known procedures. For example, 1-chloro-1,2-difluoroethylene and 1,2-dichloro-difluoroethylene are prepared by reduction, with zinc in an alcohol solution, of 1,2-difluoro-1,1,2-trichloroethane and 1,2-difluoro-tetrachloroethane, respectively (J. Chem. Soc. London 1957, pages 2800-06). The high addition of zinc necessary to the reaction and its difficult removal render these processes little attractive from an industrial viewpoint.

The preparation of 1,2-difluoroethylene using 1,2-difluoro-tetrachloroethane as a starting material is described in literature. Said reaction occurs in two steps, the former comprising the obtainment of 1,2-difluoro-1,2-dichloroethane through reduction by means of LiAlH$_4$ or through UV-radiation in the presence of 2-propanol, while the latter consists in the dehalogenation of the abovesaid intermediate in the presence of Mg (Collection Czechloslov. Chem. Commun. Vol. 39, (1974) pages 2801-07).

EP-A-53 657 describes a process for producing chlorotrifluoroethylene or trifluoroethylene by the dechlorination in the vapor phase of 1,1,2-trichloro-1,2,2-trifluoroethane or chlorotrifluoroethylene with hydrogen in the presence of a platinum-group metal supported on an alkali magnesium fluoride.

US-A-2,704,775 is directed to the preparation of chlorotrifluoroethylene by heating a mixture of 1,1,2-trichloro-1,2,2-trifluoroethane and hydrogen in the presence of a nickel catalyst.

US-A-2,864,873 relates to a process for preparing 1,1,2-trifluoro-2-chloroethylene by reacting a mixture of hydrogen and 1,1,2-trifluoro-1,2,2-trichloroethane in the vapor phase under heating and in the presence of a catalyst comprising copper metal and an oxide of chromium.

US-A-3,505,417 describes the dehalogenation of fluorohalocarbons having from 2 to 8 carbon atoms by contacting them, in the presence of hydrogen, with a catalytic composition consisting essentially of aluminium fluoride and from 0.05 to 30 weight percent of at least one of CuO, Cr$_2$O$_3$, RhCl$_3$, CoO and Pt.

US-A-3,636,173 also deals with the dehalogenation of fluorohalocarbons with hydrogen. In this case the catalyst to be employed is a nickel or chromium phosphate supported on certain forms of fluorinated alumina.

Finally, JP-A-60-185,734 (Chem. Abstr. 104, 109005x) describes the preparation of chlorotrifluoroethylene by dechlorination of 1,1,2-trichloro-1,2,2-trifluoroethane with hydrogen in the presence of active carbon from coconut shell.

It has now surprisingly been found that hydrogen-containing fluoroethylenes and chlorofluoroethylenes are preparable in a single step by reaction, in the gas phase, of certain chlorofluoroethanes with hydrogen, in the presence of certain hydrogenation catalysts.

Thus, the object of the present invention is a continuous process for preparing fluoroethylenes and chlorofluoroethylenes containing one or more hydrogen atoms, which process comprises reacting hydrogen in a single step with a chlorofluoroethane selected from 1,2-difluorotetrachloroethane, 1,1-difluorotetrachloroethane and mixtures thereof, in the gas phase at a temperature of from 200°C to 400°C, with a molar ratio of hydrogen to said chlorofluoroethane of from 2 to 5 and in the presence of a hydrogenation metal catalyst selected from palladium and nickel.

The process of the present invention is an improvement as compared with those of the art because of its profitability, easy operability on a commercial scale and because it permits to prepare hydrogen-containing fluoroethylenes and chlorofluoroethylenes in a single step.

Said process permits to obtain a very high conversion of the reagents.

The hydrogenation catalysts employed according to the present invention can be utilized either as such or, preferably, on inert materials such as, e.g., carbon, aluminium oxide, barium sulphate at a concentration ranging from 0.1 to 10% by weight.

The hydrogenation is carried out in tubular reactors made of materials such as, for example, nickel, Inconel, stainless steel.

The present process can be carried out either at atmospheric pressure or at a higher pressure.

The contact time of the reagents with the catalyst generally ranges from 5 to 60 seconds, preferably from 10 to 20 seconds.

Hydrogen can be fed either in the pure state or diluted with an inert gas such as for example nitrogen, helium, argon.

EP 0 253 410 B1

The specific molar ratio between hydrogen and chlorofluoroethanes is selected as a function of the product to be obtained; the higher said molar ratio is, the more the product mixture gets rich in hydrogenated fluoroethylenes. Furthermore, when high hydrogen/chlorofluoroethane molar ratios are employed, it is possible also to obtain the saturated products resulting from the addition of hydrogen to the fluoro-olefins and chlorofluoro-olefins produced, such as for example CFHCl-CFHCl and $CH_2F-CH_2F$.

When leaving the reactor, the vapours are washed with a water solution containing 5-20% by weight of alkali hydroxide, then they are dried over $CaCl_2$ and cooled until complete condensation.

For a better understanding of the present invention, the following illustrative examples are given, which, however, are not to be construed as a limitation of the invention.

In the examples, the percentages are by weight, unless otherwise specified.

EXAMPLES 1-10

Into a cylindrical reactor made of steel AISI 316, having an inside diameter of 12 mm and a useful volume of 125 ml, containing 100 ml of activated carbon granules with a palladium content of 0.5% by weight, there was introduced, at atmospheric pressure, a mixture, preheated to 105°C, of hydrogen and difluorotetrachloroethane. The difluorotetrachloroethane consisted of a mixture containing 88% by weight of 1,2-difluorotetrachloroethane and 12% by weight of 1,1-difluorotetrachloroethane. Contact time, reaction temperature and $H_2/C_2Cl_4F_2$ molar ratio are indicated in Table 1.

When leaving the reactor, the vapours were washed with a 10% NaOH solution, dried and condensed in a trap maintained at -78°C by means of dry ice.

According to gas-chromatographic analysis, the reaction mixture was composed of:
- difluoroethylene ($C_2H_2F_2$)
- chlorodifluoroethylene ($C_2HClF_2$)
- dichlorodifluoroethylene ($C_2Cl_2F_2$).

The percentages by weight of the products are reported in Table 1.

The balance to 100 consisted of low boiling by-products. Generally, the unreacted starting product was present only in small amounts.

EXAMPLE 11 (comparative test)

The reaction was carried out according to the modalities of Examples 1-10, with a $H_2$/reagents molar ratio equal to 5.0, a contact time of 20 seconds and a temperature of 120°C. The following results (% by weight) were obtained:

| | |
|---|---|
| $C_2H_2F_2$ | 10 |
| $C_2Cl_2F_2$ | 10 |

The balance to hundred consisted of traces of by-products and of unreacted chlorofluoroethane (78%).

EXAMPLE 12

Into a cylindrical reactor made of steel AISI 316, having an inside diameter of 12 mm and a useful volume of 125 ml, containing 100 ml of activated carbon granules, with a palladium content of 0.5% by weight, thermoregulated at 320°C, there were introduced, at atmospheric pressure, 0.37 moles/h of a mixture, preheated to 105°C, composed of hydrogen and of 1,1-difluorotetrachloroethane in the following molar ratio: $H_2/C_2Cl_4F_2$ = 2.5.

The vapours leaving the reactor were washed with a NaOH solution at 10%, dried and condensed in a trap maintained at -78°C by means of dry ice.

On gas-chromatographic analysis, the reaction mixture exhibited the following composition:

3

|                    | (% by weight) |
| ------------------ | ------------- |
| $CF_2 = CCl_2$     | 15.2          |
| $CF_2 = CHCl$      | 22.3          |
| $CF_2 = CH_2$      | 37.8          |
| $CH_3 — CHF_2$     | 9.2           |

EXAMPLE 13

Into the same reactor and under the same conditions as in Example 12 there were introduced, at atmospheric pressure, 0.37 moles/h of a mixture, preheated to 105°C, of hydrogen and of 1,2-difluorotetrachloroethane in a molar ratio $H_2/C_2Cl_4F_2 = 2.5$.

The vapours leaving the reactor were washed with a 10% NaOH solution, dried and condensed in a trap maintained at -78°C by means of dry ice. On gas-chromatographic analysis, the reaction mixture exhibited the following composition (% by weight):

| $CClF = CClF$   | 9.8  |
| --------------- | ---- |
| $CHF = CHF$     | 53.2 |
| $CClF = CHF$    | 8.4  |
| $CH_2F = CH_2F$ | 8.6  |

The balance to hundred consisted of low-boiling by-products.

EXAMPLE 14 (comparative test)

Into the same reactor and under the same conditions as in Example 12 there was introduced, at atmospheric pressure, a mixture of hydrogen and of 1,1,2-trifluorotrichloroethane in the molar ratio: $H_2/C_2Cl_3F_3 = 2.4$, said mixture having been preheated to 105°C. The contact time was of 17 seconds. The vapours leaving the reactor were washed with a 10% NaOH solution, dried and condensed in a trap maintained at -78°C by means of dry ice. The reaction mixture, subjected to gas-chromatographic analysis, exhibited the following composition:

|                  | (% by weight) |
| ---------------- | ------------- |
| $CF_2 = CHF$     | 30            |
| $CF_2 = CClF$    | 8             |
| $CF_2H - CH_2F$  | 35            |

EXAMPLES 15-17

Under the same conditions as in Example 12, but using as catalyst Ni on carbon, there was introduced into the usual reactor, at atmospheric pressure, a mixture, preheated to 105°C, composed of hydrogen and of difluorotetrachloroethane (88% by weight of 1,2-difluorotetrachloroethane and 12% by weight of 1,1-difluorotetrachloroethane).

The contact time was of 20 seconds. Three tests were carried out, using different $H_2$/difluorotetrachloroethane molar ratios. The vapours leaving the reactor were treated in like manner as in Example 12.

The gas-chromatographic analysis revealed that the reaction products were present in the % by weight reported in Table 2.

The balance to hundred consisted of low-boiling by-products.

EXAMPLE 18 (comparative test)

A test was carried out in the same reactor and under the same operative conditions of Examples 15-17, but using Cr on carbon as catalyst.

The gas-chromatographic analysis revealed that in the product mixture the olefin $C_2Cl_2F_2$ was present at 64% by weight.

Table 1

| Example | T °C | Molar ratio | Contact time (sec) | $C_2H_2F_2$ | $C_2HClF_2$ | $C_2Cl_2F_2$ | $C_2H_4F_2$ |
|---------|------|-------------|--------------------|-------------|-------------|--------------|-------------|
| 1 | 220 | 2.5 | 22 | 51 | 5.2 | 10 | 8,1 |
| 2 | 220 | 3.6 | 20 | 41 | 3.5 | 10 | 24 |
| 3* | 220 | 1.3 | 20 | 13 | 20 | 47 | 2 |
| 4 | 320 | 2.5 | 21 | 55.5 | 6.5 | 13.3 | 6.5 |
| 5 | 320 | 4.2 | 20 | 41 | 5.0 | 5.0 | 25 |
| 6* | 320 | 1.7 | 20 | 11 | 30 | 40.0 | 4.0 |
| 7 | 220 | 5.0 | 20 | 29 | 2.0 | 3 | 43 |
| 8 | 400 | 2.5 | 20 | 61 | 5.0 | 2.2 | 12.3 |
| 9 | 320 | 3.0 | 20 | 51 | 7 | 7 | 11 |
| 10* | 150 | 2.5 | 20 | 15 | 10 | 32 | 3.5 |

\* = comparative example

Table 2

| Example | Molar ratio | $C_2H_2F_2$ | $C_2HClF_2$ | $C_2Cl_2F_2$ | $C_2H_2Cl_2F_2$ |
|---------|-------------|-------------|-------------|--------------|-----------------|
| 15 | 5.0 | 80 | - | - | 7 |
| 16 | 2.5 | 45 | 37 | - | 1.5 |
| 17* | 1.3 | 5 | 24 | 62 | 1.2 |

\* = comparative example

## Claims

1. A continuous process for preparing fluoroethylenes and chlorofluoroethylenes containing one or more hydrogen atoms, which process comprises reacting hydrogen in a single step with a chlorofluoroethane selected from 1,2-difluorotetrachloroethane, 1,1-difluorotetrachloroethane and mixtures thereof, in the gas phase at a temperature of from 200°C to 400°C, with a molar ratio of hydrogen to said chlorofluoroethane of from 2 to 5 and in the presence of a hydrogenation metal catalyst selected from palladium and nickel.

2. The process according to claim 1, wherein the hydrogenation catalyst is supported on active carbon.

## Revendications

1. Un procédé continu pour préparer des fluoroéthylènes et des chlorofluoroéthylènes contenant un ou plusieurs atomes d'hydrogène, lequel procédé comprend la réaction d'hydrogène, en une seule étape, avec un chlorofluoroéthane choisi parmi le 1,2-difluorotétrachloroéthane, le 1,1-difluorotétrachloroéthane et leurs mélanges, en phase gazeuse à une température de 200°C à 400°C, avec un rapport molaire de l'hydrogène à ce chlorofluoroéthane de 2 à 5 et en présence d'un catalyseur métallique d'hydrogénation choisi parmi le palladium et le nickel.

2. Le procédé suivant la revendicaiton 1, dans lequel le catalyseur d'hydrogénation est supporté sur du carbone actif.

## Patentansprüche

1. Kontinuierliches Verfahren zur Herstellung von Fluorethylenen und Chlorfluorethylenen, die ein oder mehr Wasserstoffatome enthalten, welches umfaßt die einstufige Reaktion von Wasserstoff mit einem Chlorfluorethan, ausgewählt unter 1,2-Difluortetrachlorethan, 1,1-Difluortetrachlorethan und Mischungen davon, in der Gasphase bei einem Temperatur von 200 bis 400°C in einem Molverhältnis von Wasserstoff zu Chlorfluorethan von 2 bis 5 und in Gegenwart eines Hydrier-Metallkatalysators, ausgewählt unter Palladium und Nickel.

2. Verfahren nach Anspruch 1, worin der Hydrierkatalysator einen Aktivkohle-Träger aufweist.